# EUROPEAN PATENT APPLICATION

(11) **EP 1 741 723 A1**
(43) Date of publication of application: **10.01.2007**
(21) Application number: 05730698.7
(22) Date of filing: 08.04.2005
(51) Int. Cl.: C08B 37/08, A61K 47/36

(54) **ß-CHITIN COMPLEX AND METHOD FOR PRODUCING THE SAME**

(30) Priority: 28.04.2004 JP 2004132880
(71) Applicant: YAMAHA HATSUDOKI KABUSHIKI KAISHA, Iwata-shi, Shizuoka 438-8501 (JP); Kuga, Shigenori, Kokubunji-shi Tokyo, 1850032 (JP)
(72) Inventor: KUGA, Shigenori, 1850032 (JP); NISHIYAMA, Yoshiharu, 1610034 (JP); WADA, Masahisa, 2701424 (JP); NOISHIKI, Yasutomo, 2060034 (JP)
(74) Representative: Schlich, George William
(86) International application number: PCT/JP2005/007349
(87) International publication number: WO 2005/105853

(57) **Abstract**

The present invention provides a β-chitin complex composed of an inclusion compound formed by using a β-chitin as a host and a guest compound which is a compound having a functional group that can form a hydrogen bond with a hydroxyl group and/or an amide group of the β-chitin and having a melting point of at least 60°C. The β-chitin complex is prepared by a method comprising the step of heating a guest compound to its melting point or higher to prepare a melt of the guest compound and the step of immersing an anhydrous β-chitin in this melt, or a method comprising the step of dissolving or suspending a guest compound in a solvent to prepare a guest carrier solution and the step of immersing a β-chitin in the guest carrier solution.

## Description

### Technical Field

The present invention relates to a β-chitin complex and a method for producing the same. More specifically, the present invention relates to a β-chitin complex composed of an inclusion compound formed by using a β-chitin as a host and a particular compound as a guest compound, and allowing the guest compound to be included in between chitin molecular chain sheets in the crystal structure of the β-chitin, and a method for producing the β-chitin complex.

### Background Art

In the fields of pharmaceuticals, agricultural chemicals and other such fields, a system for delivering a required amount of an active component to a required site when required, i.e, a drug delivery system, has been widely investigated. For example, in the field of pharmaceuticals, drug delivery systems based on pharmaceutical technology, such as sustained-release preparations, timed-release preparations, and targeting preparations, have been known. The sustained-release preparations are prepared so that the release rate of drugs is controlled by means of various dosage forms such as granules, tablets, and capsules. The timed-release preparations are prepared so that active components are released at a predetermined time after administration. As the targeting preparations, colon-targeting preparations, gastroretentive preparations, and other such preparations have been known. The colon-targeting preparations are prepared so that drugs are released in the colon for the purpose of topical treatment for inflammatory colon diseases, and for other purposes.

Moreover, cyclodextrin complexes also have been known as drug delivery systems. In such a complex, cyclodextrin is used as a host and, for example, physiologically active substances susceptible to oxidation and hydrolysis, such as prostaglandin and fat-soluble vitamins, pharmaceuticals having high solubility or hygroscopicity, or other volatile substances are included as a guest in the cavity of the hydrophobic region of cyclodextrin.

However, the drug delivery systems based on pharmaceutical technology are limited because these systems depend on the dosage forms of pharmaceuticals, agricultural chemicals, or other chemicals to be delivered, the host materials to be used, and the like. Since inclusion compounds such as the cyclodextrin complexes as described above include a guest compound within a molecular-scale space of a host (e.g., cyclodextrin), it is necessary that the chemical properties of the guest compound to be included are compatible with that of the host, and furthermore that the size, shape, and other such characteristics of the guest compound fit this space. Therefore, it is very difficult to find the optimal host for individual drugs such as pharmaceuticals and agricultural chemicals that have a wide variety of chemical properties, sizes, shapes, and other such properties to form an inclusion compound. Thus, there has been a demand for a system with which a new inclusion compound can be formed.

As the host for forming a new inclusion compound, β-chitin (β-type crystal of β-1,4-poly-N-acetyl-D-glucosamine) having a structure in which sheet-like chitin molecular chains are layered has gained attention. With regard to β-chitin, Biopolymers, vol. 7, p. 281 (1969) reports the phenomenon of inclusion of water, and ADVANCES IN CHITIN SCIENCE, vol. 2, pp. 507-512 (1997) reports crystallosolvates of various linear alcohols (methanol, ethanol, n-propanol, n-butanol, and n-octanol). The present inventors have previously studied inclusion of a guest compound in between the sheets of β-chitin, and succeeded in forming β-chitin complexes composed of inclusion compounds containing acrylic acid, a linear monoamine having 3 to 9 carbon atoms, or a linear diamine having 2 to 10 carbon atoms as the guest compound (for example, Chitin and Chitosan Research, vol. 8 (No. 2), p. 186 (2002), Chitin and Chitosan Research, vol. 9 (No. 2), p. 100 (2003), and Biomacromolecules, vol. 4, pp. 944-949 (2003)).

However, according to the above-mentioned documents, there are problems in that the guest compound to be included in β-chitin is limited or the method for producing the inclusion compound is limited, for example.

### Disclosure of Invention

Going beyond these past findings, the present inventors have studied thoroughly whether or not each one of various additional compounds can be included in β-chitin. As a result, the present inventors have determined that an inclusion compound (chitin complex) can be formed when a compound having a functional group that can form a hydrogen bond with a hydroxyl group and/or an amide group of a β-chitin is included as the guest compound in between the chitin molecular chain sheets that form the stacked structure of the β-chitin, and have provided a method for producing the new inclusion compound (chitin complex), and thus achieved the present invention.

The present invention provides a β-chitin complex composed of an inclusion compound formed by using a β-chitin as a host and a guest compound which is a compound having a functional group that can form a hydrogen bond with a hydroxyl group and/or an amide group of the β-chitin, and having a melting point of at least 60°C.

In an embodiment, the guest compound is an organic compound or an organometallic compound.

In another embodiment, the functional group has at least one atom selected from the group consisting of oxygen, nitrogen, sulfur, and halogen atoms.

In still another embodiment, the functional group is at least one functional group selected from the group consisting of a hydroxyl group, an aldehyde group, a carboxyl group, a carbonyl group, an ether bond, an ester bond, a ketal bond, an amino group, an amidino group, an imino bond, a diazo bond, an amide group, an amide bond, an imino ether bond, a mercapto group, a thiocarbonyl group, a thioaldehyde group, a thioester group, a thioether bond, a thioketal bond, and a halogenated alkyl group.

In yet another embodiment, the guest compound has a plurality of functional groups.

In a different embodiment, the guest compound is a saccharide having a plurality of hydroxyl groups.

In still another embodiment, the guest compound is at least one compound selected from the group consisting of vitamins, coenzymes, hormones, antibiotics, neurotransmitters, intercellular mediators, immune reaction inhibitors, immune reaction accelerators, enzyme inhibitors, organic physiologically active substances, insecticides, antibacterial agents, aromatics, spices, and seasoning agents.

Moreover, the present invention provides a method for producing a β-chitin complex composed of an inclusion compound containing a β-chitin as a host, which includes heating a guest compound to the melting point or to a temperature higher than the melting point but lower than 250°C to prepare a melt of the guest compound; and immersing an anhydrous β-chitin in the melt, wherein the guest compound has a functional group that can form a hydrogen bond with a hydroxyl group and/or an amide group of a β-chitin and has a melting point of at least 60°C.

Furthermore, the present invention provides a method for producing a β-chitin complex composed of an inclusion compound containing a β-chitin as a host, which includes dissolving or suspending a guest compound having a functional group that can form a hydrogen bond with a hydroxyl group and/or an amide group of a β-chitin in a solvent to prepare a guest carrier solution; and immersing a β-chitin in the guest carrier solution.

In an embodiment, the solvent is a poor solvent for the guest compound.

In another embodiment, the solubility of the guest compound in the solvent is 5 wt% or less.

In still another embodiment, the solvent is an organic solvent.

The present invention further provides a method for producing a β-chitin complex composed of an inclusion compound containing a β-chitin as a host, which includes allowing an undesired guest compound to be included in a β-chitin in advance; and adding a desired guest compound so as to substitute the same for the undesired guest compound, thereby allowing the desired guest compound to be included in the β-chitin.

In an embodiment, the substitution of the desired guest compound for the undesired guest compound is performed by a process including a step of dissolving or suspending a desired guest compound in a solvent to prepare a guest carrier solution; and a step of immersing a β-chitin including the undesired guest compound in the guest carrier solution.

### Brief Description of Drawings

Fig. 1 is a chart showing the X-ray diffraction data of a β-chitin as well as a β-chitin complex C and β-chitin complexes E to G obtained in Example 3 and Examples 5 to 8 of the present invention.
Fig. 2 is a chart showing the X-ray diffraction data when pyromellitic dianhydride is used as the guest compound.

### Best Mode for Carrying Out the Invention

### β-Chitin

Chitin, β-1,4-poly-N-acetyl-D-glucosamine, includes α-chitin having an α-type crystal structure, β-chitin having a β-type crystal structure, and other such chitins. In the present invention, a β-chitin having the β-type crystal structure is used. In β-chitin, chitin molecular chains, all of which are parallel to one another and are oriented in the same direction, form sheets by Van der Waals force and hydrogen bonding between the chitin molecular chains. β-Chitin has a structure in which the sheets are layered by hydrophobic bonding (sheet-like stacked structure). Since bonding between the sheets is much weaker than bonding within the sheets, a certain type of low-molecular substance can be inserted between the chitin sheets upon having made contact with the β-chitin crystal, and thus form a relatively stable inclusion compound.

There is no particular limitation regarding the molecular weight of the β-chitin used in the present invention. The degree of polymerization of the β-chitin is preferably at least 50, more preferably at least 100, and even more preferably at least 500 or at least 1000. When the degree of polymerization is too high, handling tends to be difficult, so that the degree of polymerization is preferably not more than 2000. β-Chitin obtained by homogenizing a cultivated algae product has a degree of polymerization of approximately 1000 and is preferably used.

In the β-chitin used in the present invention, a portion of the acetyl groups thereof may be eliminated (partially deacetylated chitin).

It is also possible to use a commercially available β-chitin. Alternatively, it is possible to use β-chitin obtained by cultivating an alga (diatom) belonging to the genus *Thalassiosira* and collecting and purifying the produced β-chitin. Although β-chitin generally exists as a hydrate, an anhydrous β-chitin may be used.

It is also possible to form a complex of β-chitin and use this complex as the host. Preferred examples of a substance that can form a complex with β-chitin include hexylamine, water, ethanol, and dimethylsulfoxide.

### Guest compound

As the guest compound in the present invention, a compound having a functional group that can form a hydrogen bond with a hydroxyl group and/or an amide group of the β-chitin is used. As the guest compound, a compound having a melting point of at least 60°C is preferable, and a compound having a melting point of at least 100°C is more preferable. A compound having a melting point of lower than 60°C is not useful for forming an inclusion compound with the β-chitin to produce a β-chitin complex.

In view of the ability to be strongly bonded to chitin molecules, the functional group of the guest compound preferably has at least one atom selected from the group consisting of oxygen, nitrogen, sulfur, and halogen atoms, which have at least one unshared electron pair. Examples of the functional group having such an atom include a hydroxyl group, an aldehyde group, a carboxyl group, a carbonyl group, an ether bond, an ester bond, a ketal bond, an amino group, an amidino group, an imino bond, a diazo bond, an amide group, an amide bond, an imino ether bond, a mercapto group, a thiocarbonyl group, a thioaldehyde group, a thioester group, a thioether bond, a thioketal bond, and a halogenated alkyl group. The guest compound contains one or more such functional group. In view of the affinity with chitin molecules, the guest compound preferably has two or more such functional groups.

The guest compound is preferably an organic compound or an organometallic compound, but may be an inorganic compound.

Examples of the organic compound to be used as the guest compound are: vitamins such as vitamin C (ascorbic acid), vitamin Bx (p-aminobenzoic acid which is a vitamin B complex), vitamin B12 (cyanocobalamin), and vitamin PQQ (pyrroloquinoline quinone); coenzymes such as nicotinamide, biocytin, and thiamin diphosphate; hormones such as epinephrine (an adrenal hormone), estrone (a steroidal estrogenic hormone), and progesterone (a steroidal corpus luteum hormone); antibiotics such as peplomycin sulfate and mitomycin C; neurotransmitters such as indomethacin (a nonsteroidal antiinflammatory drug) and diazepam (an antianxiety drug, a hypnotic, a muscle relaxant, an anticonvulsant); intercellular mediators such as cytokines and chemokines; immune reaction inhibitors such as methotrexate (an antimetabolite anticancer drug) and flutamide (a hormonal anticancer drug); immune reaction accelerators such as interleukins; enzyme inhibitors such as an angiotensin converting enzyme inhibitor; organic physiologically active substances such as spermine that activates a nucleic acid biosynthesis system and a protein synthesis system; agricultural chemicals; antibacterial substances such as agricultural or household insecticides, bactericides, antibacterial agents, and fungicides; aromatics; spices; and seasoning agents.

Examples of the organometallic compound include carboplatin (a platinum complex-based anticancer drug) and nedaplatin (a platinum complex-based anticancer drug).

Examples of the inorganic compound include cisplatin (a platinum complex-based anticancer drug).

It is also possible to use two or more of the above-mentioned compounds in combination for the same purpose or to obtain a plurality of effects. Method for producing the inclusion compound

Examples of the method for allowing the guest compound to be included in the β-chitin include a method of immersing the β-chitin in a melt of the guest compound (immersing-in-melt method), a method of immersing the β-chitin in a solution or a dispersion of the guest compound (immersing-in-solution method), and a method of allowing an undesired guest compound to be included temporarily in the chitin to increase the spacing between the chitin chain sheets of the β-chitin, and then adding a desired guest compound so as to substitute it for the undesired guest compound, thereby allowing the desired compound to be included (guest substitution method).

### 1. Immersing-in-melt method

This method is used when the guest compound has a melting point of at least 60°C and also the guest compound is thermally stable even when heated to its melting point or higher and easily becomes a melt. The method includes the step of heating the guest compound to a temperature of at least the melting point of the guest compound and at most the degradation temperatures of the guest compound and β-chitin so as to provide a melt, and the step of immersing an anhydrous β-chitin in the obtained melt. At a temperature of 250°C or higher, β-chitin is carbonized easily. Therefore, when using this method, it is preferable that the guest compound have a melting point of not higher than 250°C. More preferably, the melting point is not higher than 200°C and still more preferably not higher than 150°C. This method has an advantage in that the guest compound can be brought into contact with the β-chitin, which is the host, directly and at high concentrations and thus a complex can be formed quickly.

In this method, the β-chitin may be an anhydride or a hydrate. Generally, β-chitin exists as a hydrate. An anhydrous β-chitin can be obtained by drying a β-chitin hydrate at 105°C for 2 hours.

### 2. Immersing-in-solution method

This method includes the step of dissolving or suspending the guest compound in a solvent to prepare a solution of the guest compound (guest carrier solution), and the step of immersing the β-chitin in the guest carrier solution so as to allow the guest compound to be included in the β-chitin. The solvent to be used is required not to be reactive with the guest compound and the β-chitin, and to be a solvent that does not dissolve the β-chitin. The guest carrier solution may be a solution in which the entire guest compound has completely been dissolved in the solvent, or may be a suspension in which a portion of the guest compound has been dissolved in the solvent.

The concentration of the guest compound in the guest carrier solution may be varied depending on the combinations of the guest compound and solvent to be used. There is no limitation regarding the solubility of the guest compound in the solvent to be used. A solvent in which the guest compound is dissolved easily (easily-dissolving solvent) or a solvent in which the guest compound is slightly dissolved (slightly-dissolving solvent) may be used.

The lower the solubility of the guest compound, the more easily the guest carrier solution is saturated and thus the more easily the guest compound is included in the β-chitin. Therefore, a slightly-dissolving solvent is preferably used in order to allow the guest compound to be included in the β-chitin efficiently. The concentration of the guest compound dissolved in the solvent is preferably not more than 5.0 wt% and more preferably in a range from 0.2 to 2.0 wt%.

Furthermore, by using the guest carrier solution in the form of a suspension, the following advantage is provided. That is, when the dissolved guest compound is included in the β-chitin, the guest compound in the suspension dissolves in the solvent and thus the saturated state is maintained in the solvent. Therefore, the guest compound is included in the β-chitin more efficiently.

As described above, the method of forming a β-chitin complex by reacting the guest compound with the β-chitin using a solvent that is a poor solvent for the guest compound is particularly useful when a rare physiologically active substance or other such substance is used as the guest compound. The reason is that by employing this method, the advantages are that only a small amount of the guest compound is used and that any loss during the production process can be reduced as much as possible. Furthermore, with this method the amount of the guest compound to be included can be controlled, so that when the inclusion compound is a drug, the amount of the drug can be controlled. Therefore, this method is useful particularly for pharmaceutical drug delivery systems. Moreover, this method is not affected by the melting point of the guest compound. Therefore, this method is effective for inclusion of a substance having such a high melting point that it cannot be used in the above-described immersing-in-melt method, for example, pyromellitic dianhydride (melting point: 286°C) and other such substances.

The solvent used in this immersing-in-solution method can be selected appropriately according to the nature, solubility, and other such properties of the guest compound. Such a solvent includes water; monohydric or polyhydric alcohols such as methanol, ethanol, isopropanol, ethylene glycol, and glycerin; ketones such as acetone and methyl ethyl ketone; aromatic hydrocarbons such as benzene, toluene, xylene, and pyridine; aliphatic hydrocarbons such as n-hexane and cyclohexane; halogenated hydrocarbons such as monochloromethane, dichloromethane, and chloroform; amines such as methylamine, ethylamine, and ethylenediamine; organic acids such as formic acid, acetic acid, and propionic acid; nitriles such as acetonitrile and propionitrile; and aprotic polar solvents such as N,N'-dimethylformamide, N,N'-dimethylacetamide, N,N'-dimethyl sulfoxide, and N,N'-dimethylimidazolidine.

### 3. Guest substitution method

This method includes the following two steps: a step of first allowing an undesired guest compound to be included in the β-chitin, and a step of then adding a desired guest compound so as to substitute it for the undesired guest compound, thereby allowing the desired compound to be included in the β-chitin. The above-described immersing-in-melt method or immersing-in-solution method can be applied to these two steps independently.

This method may be a multi-stage guest substitution method in which these two steps are repeated. For example, it is also possible that an undesired guest compound that is easiest to include is first included, then an undesired guest compound that is the next easiest to include is substituted for that undesired guest compound, and finally a desired guest compound is included by substitution. In particular, when the immersing-in-solution method is used to substitute the desired guest compound for the undesired guest compound, the desired guest compound can be substituted for the undesired guest compound more efficiently by using a solvent that can relatively easily dissolve the undesired guest compound but only slightly dissolves the desired guest compound.

Regarding this multi-stage guest substitution method, it is preferable to select and use the successive undesired guest compounds so as to increase incrementally the spacing between the sheets, and finally substitute the desired guest compound for the last used undesired guest compound.

### β-Chitin complex

The β-chitin complex of the present invention is an inclusion compound having β-chitin as the host and various compounds as the guest compound. This β-chitin complex (inclusion compound) is an intercalation compound in which the guest compound is interposed between the chitin chain sheets that form the crystal lattice of chitin. Regarding the β-chitin complex having such a structure, when the guest compound is easily dissolved in a surrounding liquid, the rate at which the readily soluble guest compound is dissolved and is diffused in the surrounding liquid can be controlled by this β-chitin complex. On the other hand, when the guest compound is slightly soluble in a surrounding liquid and is thus difficult to administer in the form of a solution or a suspension, the β-chitin complex can be formed by forming β-chitin into a powder, film, fibrous product, or other such forms and allowing the β-chitin to include the guest compound. When such a β-chitin complex is supplied to the body, an unwanted side effect such as dissipation or irritation to tissues can be suppressed. Thus, it is believed that the β-chitin complex is very useful in terms of constructing a drug delivery system using, for example, a physiologically active substance as the guest compound. Therefore, the β-chitin complex of the present invention is useful as a pharmaceutical and an agricultural chemical as well as a bactericide, a fungicide, an insecticide, a food stuff, an aromatic, and other such products for agricultural, household, and other uses.

### Examples

Hereinafter, the present invention will be described specifically by way of examples. However, the present invention is not limited to these examples.

### Preparation example: Preparation of β-chitin

The centric diatom *Thalassiosira weissflogii* (CCMP 1051) strain was used. This strain was inoculated into an artificial seawater medium (f/2 medium) and cultured for 2 weeks at 25°C under aeration and illumination at 5000 lux. The composition of the f/2 medium is shown in Table 1 below.

**Table 1**

| Compound | Concentration of stock solution | Amount used |
|---|---|---|
| NaNO₃ | 75.0 g/L H₂O | 1.0 ml |
| NaH₂PO₄ | 5.0 g/L H₂O | 1.0 ml |
| Na₂SiO₃ | 30.0 g/L H₂O | 1.0 ml |
| f/2 trace metal solution | Note 1 | 1.0 ml |
| f/2 vitamin solution | Note 2 | 0.5 ml |
| adjusted to 1 L with filtered seawater | | |
| Note 1: f/2 trace metal solution | | |

| Inorganic metal | Concentration of stock solution | Amount used |
|---|---|---|
| FeCl₃ · 6H₂O | - | 3.15 g |
| Na₂EDTA · 2H₂O | - | 4.36 g |
| CuSO₄ · 5H₂O | 9.8 g/L H₂O | 1.0 ml |
| Na₂MO₄ · 2H₂O | 6.3 g/L H₂O | 1.0 ml |
| ZnSO₄ · 7H₂O | 22.0 g/L H₂O | 1.0 ml |
| CoCl₂ · 6H₂O | 10.0 g/L H₂O | 1.0 ml |
| MnCl₂ · 4H₂O | 180.0 g/L H₂O | 1.0 ml |
| adjusted to 1 L with filtered seawater | | |
| Note 2: f/2 vitamin solution | | |

| Vitamin | Concentration of stock solution | Amount used |
|---|---|---|
| Vitamin B12 | 1.0 g/L H₂O | 1.0 ml |
| Biotin | 0.1 g/L H₂O | 10.0 ml |
| Thiamine hydrochloride | - | 200 mg |
| adjusted to 1 L with filtered seawater | | |

The resultant culture of *Thalassiosira* was mechanically shaken to eliminate β-chitin spines from the algae. After shaking, the culture was centrifuged at 1000 × g, and the supernatant was subjected to decantation to collect β-chitin. The obtained crude β-chitin was dispersed in 5 wt% aqueous potassium hydroxide, and allowed to stand at room temperature for 12 hours. Then, the β-chitin was collected from the potassium hydroxide solution by filtration. The collected β-chitin was redispersed in methanol, and maintained at 60°C for 2 hours. The β-chitin was separated by filtration again, boiled with 0.1 mol/L aqueous hydrochloric acid for one hour, then dispersed in 0.34 wt% aqueous sodium chlorite, and maintained at 80°C for 2 hours. The β-chitin was collected by filtration, dispersed in 1 wt% aqueous hydrofluoric acid, and allowed to stand at room temperature for 12 hours. The β-chitin was collected by filtration and dried at room temperature.

The resultant purified β-chitin was dried at room temperature to give β-chitin hydrate. The β-chitin hydrate was dried at 105°C for 2 hours to prepare anhydrous β-chitin.

A sample for X-ray diffraction analysis was prepared in the following manner. First, 5 mg of the dry β-chitin were dispersed in 7 ml of water. Then, 3 ml of 1 wt% aqueous fibrinogen solution was added to this dispersion of the β-chitin, and mixed slowly. The mixture was supplemented by several drops of a concentrated aqueous thrombin solution (about 2 wt%), and then mixed and spread on a petri dish for gelation. As a result of the gelation, a gel having a thickness of about 3 mm was obtained. This gel was cut into a 5 mm x 50 mm strip, and the strip was stretched on a filter paper slowly by hand to about three times its original length while removing water. The stretched gel was immersed in 1% aqueous potassium hydroxide to loosen fibrinogen, and further stretched to from 1.5 to 2 times its length (from 4.5 to 6 times its original length). The resultant string-like sample was cut into about 2 cm pieces, and immersed in 10 ml of 5% potassium hydroxide solution. The potassium hydroxide solution was warmed and maintained at 50°C for 10 minutes, and thus fibrinogen was dissolved. Thereafter, the string-like chitin sample was immersed in 10 ml of distilled water, and KOH was removed by changing the water several times, and then the sample was dried. This chitin sample was analyzed using an X-ray diffraction analyzer.

### Example 1: Preparation of β-chitin complex A with p-phenylenediamine

First, p-phenylenediamine (melting point: 140°C) serving as the guest compound was melted by heating to 150°C. The anhydrous β-chitin prepared in the above-described preparation example was immersed in this melt for 30 minutes. Then, the β-chitin was removed from the melt, and the surface of the β-chitin was wiped quickly with a filter paper to remove the melt adhered to the external surface of the β-chitin. Thus, a β-chitin complex including p-phenylenediamine (β-chitin complex A) was obtained.

The inclusion of p-phenylenediamine was confirmed by measuring the spacing between the chitin molecular chain sheets (sheet spacing) in the crystal structure of the β-chitin by X-ray diffraction analysis.

The diffraction angle (2θ) of a peak corresponding to the sheet spacing of the anhydrous β-chitin was 9.6°. The sheet spacing (d) of the anhydrous β-chitin, which was calculated from this value using Bragg's equation (2dsinθ = λ), was 0.92 nm. The symbol λ represents the wavelength, 0.154 nm, of the X-ray used for the measurement.

On the other hand, the diffraction angle (2θ) of a peak corresponding to the sheet spacing of the β-chitin-p-phenylenediamine complex A was 7.1°. The sheet spacing (d) of the β-chitin complex A, which was calculated from this value, was 1.296 nm. From the result of this X-ray diffraction analysis, the inclusion of p-phenylenediamine was confirmed because in the β-chitin complex A, the spacing between the chitin molecular chain sheets was larger than that in the anhydrous β-chitin.

p-Phenylenediamine was extracted by immersing the β-chitin complex A in an appropriate amount of ethanol, and analyzed by gas chromatography, thereby the inclusion amount of p-phenylenediamine (guest inclusion amount) was obtained. The guest inclusion amount in the β-chitin complex A was one p-phenylenediamine molecule per chitobiose unit (guest/β-chitin = 108/406 = 0.266).

### Example 2: Preparation of β-chitin complex B with p-phenylenediamine

A guest carrier solution was prepared by dissolving p-phenylenediamine (guest compound) in benzene so as to contain p-phenylenediamine at 1 wt%. The β-chitin hydrate prepared in the above-described preparation example was immersed in this guest carrier solution at 25°C for 30 minutes, and then removed from the guest carrier solution. The surface of the β-chitin was wiped quickly with a filter paper to remove the guest carrier solution adhered to the external surface of the β-chitin, and thus a β-chitin complex including p-phenylenediamine (β-chitin complex B) was obtained.

For the obtained β-chitin complex B the sheet spacing was measured by X-ray diffraction analysis in the same manner as in Example 1. The sheet spacing of the β-chitin complex B was 1.30 nm, which was larger than the sheet spacing 0.92 nm of the anhydrous β-chitin. This result revealed that the obtained β-chitin complex B was an inclusion compound formed by the β-chitin as the host and p-phenylenediamine as the guest.

This complex B is substantially identical to the complex A of Example 1. Therefore, the method of Example 2 can provide a superior effect in that p-phenylenediamine, which is the guest compound, can be included in the β-chitin from the solution at room temperature without being heated to a temperature as high as 150°C.

### Example 3: Preparation of β-chitin complex C with p-phenylenediamine

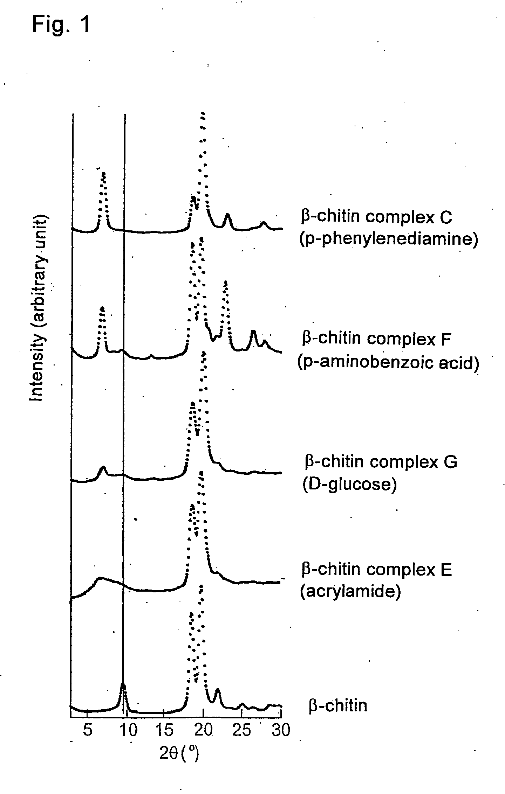

A β-chitin complex C was obtained in the same manner as in Example 2, except that ethanol was used as the solvent for preparing the guest carrier solution. Data from X-ray diffraction analysis of the β-chitin complex C is shown in Fig. 1. The sheet spacing of the β-chitin complex C was larger than that of the anhydrous β-chitin.

### Example 4: Preparation of β-chitin complex D with acrylamide

First, a guest carrier solution was prepared by dissolving acrylamide (guest compound) having a melting point of 86°C in benzene so as to contain acrylamide at 0.3 wt%. The β-chitin hydrate prepared in the above-described preparation example was immersed in this guest carrier solution for 30 minutes, and removed from the guest carrier solution. The surface of the removed β-chitin was wiped quickly with a filter paper to remove the guest carrier solution adhered to the external surface of the β-chitin, and thus a β-chitin complex including acrylamide (β-chitin complex D) was obtained.

For the obtained β-chitin complex D the sheet spacing was measured by X-ray diffraction analysis in the same manner as in Example 1. The sheet spacing of the β-chitin complex D was 1.27 nm, which was larger than that of the anhydrous β-chitin, 0.92 nm. This result revealed that the obtained β-chitin complex D was an inclusion compound containing the β-chitin as the host and acrylamide as the guest.

### Example 5: Preparation of β-chitin complex E with acrylamide

A β-chitin complex E including acrylamide was obtained in the same manner as in Example 4, except that the β-chitin complex was prepared by immersing a hexylamine complex of the β-chitin in the guest carrier solution of Example 4 maintained at 50°C. The sheet spacing of the β-chitin complex E was larger than that of the anhydrous β-chitin. Data from X-ray diffraction analysis of this β-chitin complex E is shown in Fig. 1. The hexylamine complex of the β-chitin was prepared by immersing the β-chitin in hexylamine at room temperature.

### Example 6: Preparation of β-chitin complex F with p-aminobenzoic acid

First, a guest carrier solution was prepared by dissolving p-aminobenzoic acid (guest compound) having a melting point of 186°C in benzene so as to contain p-aminobenzoic acid at 0.2 wt%. The β-chitin hydrate prepared in the above-described preparation example was immersed in this guest carrier solution at 50°C for 30 minutes, and removed from the guest carrier solution. The surface of the β-chitin removed was wiped quickly with a filter paper to remove the guest carrier solution adhered to the external surface of the β-chitin, and thus a β-chitin complex including p-aminobenzoic acid (β-chitin complex F) was obtained.

For the obtained β-chitin complex F the sheet spacing was measured by X-ray diffraction analysis in the same manner as in Example 1. The sheet spacing of the β-chitin complex F was 1.31 nm, which was larger than that of the anhydrous β-chitin, 0.92 nm. This result revealed that the obtained β-chitin complex F was an inclusion compound formed by the β-chitin as the host and p-aminobenzoic acid as the guest. Data from X-ray diffraction analysis of this β-chitin complex F is shown in Fig. 1.

### Example 7: Preparation of β-chitin complex G with α-D-glucose

First, a guest carrier solution was prepared by dissolving anhydrous α-D-glucose (guest compound) having a melting point of 146°C in pyridine so as to contain anhydrous α-D-glucose at 7 wt%. The β-chitin hydrate prepared in the above-described preparation example was immersed in this guest carrier solution at room temperature for 30 minutes, and then removed from the guest carrier solution. The surface of the removed β-chitin was wiped quickly with a filter paper to remove the guest carrier solution adhered to the external surface of the β-chitin, and thus a β-chitin complex including α-D-glucose (β-chitin complex G) was obtained.

For the obtained β-chitin complex G the sheet spacing was measured by X-ray diffraction analysis in the same manner as in Example 1. The sheet spacing of the β-chitin complex G was 1.31 nm, which was larger than that of the anhydrous β-chitin, 0.92 nm. This result revealed that the obtained β-chitin complex G was an inclusion compound formed by the β-chitin as the host and α-D-glucose as the guest. Data from X-ray diffraction analysis of this β-chitin complex G is shown in Fig. 1.

### Example 8: Preparation of β-chitin complex H with pyromellitic dianhydride

First, a saturated acetone solution of pyromellitic dianhydride (melting point: 285°C) serving as the guest compound was prepared. A hexylamine complex with the β-chitin was immersed in this saturated acetone/pyromellitic dianhydride solution for one hour. Then, the β-chitin was removed from the solution, and the surface of the β-chitin was wiped quickly with a filter paper to remove the solution adhered to the external surface of the β-chitin. Thus, a β-chitin complex including pyromellitic dianhydride (β-chitin complex H) was obtained.

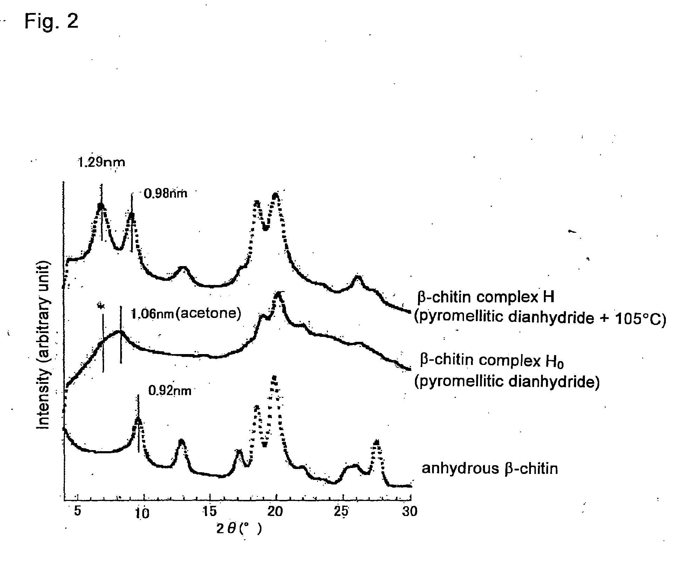

For the obtained β-chitin complex H the sheet spacing was measured by X-ray diffraction analysis in the same manner as in Example 1. The sheet spacing of the β-chitin complex H was 1.29 nm, which was larger than that of the anhydrous β-chitin, 0.92 nm. This result revealed that the obtained β-chitin complex H was an inclusion compound formed by the β-chitin as the host and pyromellitic dianhydride as the guest. Data from X-ray diffraction analysis of the β-chitin complex H is shown in Fig. 2. Data from X-ray diffraction analysis of a β-chitin complex Ho that was obtained in the case where a hexylamine complex with the β-chitin was immersed in pyromellitic dianhydride is also shown in Fig. 2.

### Industrial Applicability

The β-chitin complex of the present invention is composed of an inclusion compound that is formed by using a β-chitin as a host and using as a guest a compound having a functional group that can form a hydrogen bond with a hydroxyl group and/or an amide group of this β-chitin and having a melting point of at least 60°C. When, for example, a physiologically active substance is selected as the guest compound, a drug delivery system for this physiologically active substance can be constructed. The β-chitin complex of the present invention is very useful particularly in the field of pharmaceuticals and agricultural chemicals.

Moreover, according to the method for producing a β-chitin complex of the present invention, such a β-chitin complex can be industrially produced with ease. The method of the present invention is industrially very useful particularly in the field of pharmaceuticals and agrochemicals.

## Claims

1. A β-chitin complex comprising an inclusion compound formed by using a β-chitin as a host and a guest compound which is a compound having a functional group that can form a hydrogen bond with a hydroxyl group and/or an amide group of the β-chitin and having a melting point of at least 60°C.

2. The β-chitin complex of claim 1, wherein the guest compound is an organic compound or an organometallic compound.

3. The β-chitin complex of claim 1 or 2, wherein the functional group has at least one atom selected from the group consisting of oxygen, nitrogen, sulfur, and halogen atoms.

4. The β-chitin complex of any one of claims 1 to 3, wherein the functional group is at least one functional group selected from the group consisting of a hydroxyl group, an aldehyde group, a carboxyl group, a carbonyl group, an ether bond, an ester bond, a ketal bond, an amino group, an amidino group, an imino bond, a diazo bond, an amide group, an amide bond, an imino ether bond, a mercapto group, a thiocarbonyl group, a thioaldehyde group, a thioester group, a thioether bond, a thioketal bond, and a halogenated alkyl group.

5. The β-chitin complex of any one of claims 1 to 4, wherein the guest compound has a plurality of functional groups.

6. The β-chitin complex of any one of claims 1 to 5, wherein the guest compound is a saccharide having a plurality of hydroxyl groups.

7. The β-chitin complex of any one of claims 1 to 6, wherein the guest compound is at least one compound selected from the group consisting of vitamins, coenzymes, hormones, antibiotics, neurotransmitters, intercellular mediators, immune reaction inhibitors, immune reaction accelerators, enzyme inhibitors, organic physiologically active substances, insecticides, antibacterial agents, aromatics, spices, and seasoning agents.

8. A method for producing a β-chitin complex composed of an inclusion compound containing a β-chitin as a host, comprising:
heating a guest compound having a functional group that can form a hydrogen bond with a hydroxyl group and/or an amide group of a β-chitin and having a melting point of at least 60°C to the melting point or to a temperature higher than the melting point but lower than 250°C to prepare a melt of the guest compound; and
immersing an anhydrous β-chitin in the melt.

9. A method for producing a β-chitin complex composed of an inclusion compound containing a β-chitin as a host, comprising:
dissolving or suspending a guest compound having a functional group that can form a hydrogen bond with a hydroxyl group and/or an amide group of a β-chitin in a solvent to prepare a guest carrier solution; and
immersing a β-chitin in the guest carrier solution.

10. The method for producing a β-chitin complex of claim 9, wherein the solvent is a poor solvent for the guest compound.

11. The method for producing a β-chitin complex of claim 9 or 10, wherein the solubility of the guest compound in the solvent is 5 wt% or less.

12. The method for producing a β-chitin complex of any one of claims 9 to 11, wherein the solvent is an organic solvent.

13. A method for producing a β-chitin complex composed of an inclusion compound containing a β-chitin as a host, comprising:
allowing an undesired guest compound to be included in a β-chitin in advance; and
adding a desired guest compound so as to substitute the same for the undesired guest compound, thereby allowing the desired guest compound to be included in the β-chitin.

14. The method for producing a β-chitin complex of claim 13, wherein the substitution of the desired guest compound for the undesired guest compound is performed by a process comprising:
a step of dissolving or suspending a desired guest compound in a solvent to prepare a guest carrier solution; and
a step of immersing a β-chitin including the undesired guest compound in the guest carrier solution.
